# EUROPEAN PATENT SPECIFICATION

(11) **EP 3 806 728 B1**
(45) Date of publication and mention of the grant of the patent: **17.11.2021**
(21) Application number: 19730176.5
(22) Date of filing: 17.06.2019
(51) Int. Cl.: A61B 5/242

(54) **DEVICE AND METHOD FOR USE IN INDUCTIVE SENSING**
INDUKTIVE SENSORVORRICHTUNG UND VERFAHREN
DISPOSITIF ET PROCÉDÉ DE DÉTECTION INDUCTIVE

(30) Priority: 18.06.2018 EP 18178261
(43) Date of publication of application: 21.04.2021
(73) Proprietor: Koninklijke Philips N.V., 5656 AG Eindhoven (NL)
(72) Inventor: DOODEMAN, Gerardus, Johannes, Nicolaas, 5656 AE Eindhoven (NL); KLEIJNEN, Mark, Peter, Paul, 5656 AE Eindhoven (NL); LEIJSSEN, Jacobus, Josephus, 5656 AE Eindhoven (NL); GROSFELD, Ronny, Hubertus, Johannes, 5656 AE Eindhoven (NL); PEETERS, Wouter, Herman, 5656 AE Eindhoven (NL); BEZEMER, Rick, 5656 AE Eindhoven (NL)
(74) Representative: Philips Intellectual Property & Standards
(86) International application number: PCT/EP2019/065797
(87) International publication number: WO 2019/243213

(56) References cited:
- EP-A1- 2 275 028
- WO-A1-2016/202996
- US-A1- 2008 218 180
- US-A1- 2010 026 318
- US-A1- 2017 042 437
- US-A1- 2017 085 130

## Description

### FIELD OF THE INVENTION

The invention relates to a device for use in inductive sensing for coupling electromagnetic signals into and out of a medium.

### BACKGROUND OF THE INVENTION

Inductive sensing can be used as a means of non-invasive investigation of properties of a body.

In one advantageous area of application, inductive sensing can be used as a means of non-invasively investigating physiological characteristics, in particular heart and lung dynamics. Inductive sensing is based on magnetic induction and has several advantages over conductive and capacitive sensing.

An advantage compared to conductive sensing, such as bio-impedance measurements, is that adhesive electrodes are not required; sensing may be performed without contact and/or through non-conductive material, such as textile and plastic. In addition, inductive sensing signals are significantly less susceptible to corruption by motion artifacts.

An advantage compared with capacitive sensing is that inductive sensing is based on magnetic fields rather than electric fields and as a result is more sensitive to changes at greater penetration depth inside the body, as opposed to those just occurring at skin level. This is because magnetic fields penetrate deeper into a body than electrical fields, and thus magnetic fields can be used to measure changes in properties deeper inside the body, whereas electrical fields are predominantly useful only for measuring effects at the surface of the skin such as changes in skin properties (e.g. permittivity) or movement of the skin (skin proximity).

Coil-based inductive sensors function by inductively coupling with electromagnetic signals (i.e. electromagnetic waves or oscillations), wherein propagation of the signals through the coil leads to a change in the current through the coil, which can be measured and used to sense properties of the propagated signal (including e.g. frequency spectrum, amplitude and phase pattern).

An electromagnetic excitation signal can be propagated into a body to be investigated. The excitation electromagnetic signal causes magnetic induction in the body, i.e. the generation of eddy currents in the tissue of the body due to the application of an external magnetic field. These eddy currents then in turn generate electromagnetic signals propagated out of the body which interact with the applied fields in a way that allows them to be sensed by the coil.

Movements of tissue in the body can manifest in changes in volumes of local regions of the tissue and in changes of the conductive or dielectric properties of a tissue. These changes then cause amplitude and/or phase modulations of the electromagnetic signal which are emitted out of the body in response to the electromagnetic stimulation. By monitoring these changes, movement and size change of elements within the body can be detected and tracked, and changes in the conductivity and dielectric properties can be tracked. For example, heart contractions manifest themselves mainly as movement of blood, and breathing mainly manifests itself as changes in the conductivity of the lung.

Different structures within the body are sensitive to electromagnetic stimulation at different frequencies. This can depend in particular upon the dimensions of the structure, but also its material properties, for example its electrical conductivity properties. For example, high-frequency measurements are predominantly sensitive to the skin (and therefore also motion), while medium- and/or low-frequency signals have optimized sensitivity to the heart and/or lungs (but also are sensitive to motion). In addition, different frequencies permit stimulation at different depths within the body.

WO 2016/202996 A1, EP2275028 A1, US 2017/042437 A1, US 2008/218180 A1 and US 2017/085130 A1 each discloses an antenna for transmitting and receiving signals over predetermined frequency bands.

An inductive sensing device capable of stimulating a medium at more than one frequency is therefore of advantage.

### SUMMARY OF THE INVENTION

The invention is defined by the claims.

One solution considered by the inventors would be to use an adaptive drive mechanism which is operable to drive an antenna at different frequencies. However, it has been found that for such a device, the high frequency mode of operation is capable only of generating a weak magnetic field. This means sensing resolution is reduced.

More particularly, it has been recognized by the inventors that where a single antenna coil is operated at multiple frequencies, the higher-frequency oscillation modes are necessarily either differential modes or common modes. In both cases, an antenna operating in these modes operates as a strong (electric) dipole antenna. This behavior is undesirable for inductive sensing, since it renders the antenna highly sensitive to capacitive coupling with the body being probed. This is a surface effect which leads to generation of noise in the (target) inductive sensing signal emanating from inside the body. This reduces the strength of the sensed inductive signal and hence the accuracy of any derived measurements.

An improved inductive sensing device for sensing at multiple inductive frequencies is sought.

According to examples in accordance with an aspect of the invention, there is provided an device for use in inductive sensing, for inductively coupling electromagnetic signals into and out of a medium, the device comprising:
an antenna, the antenna comprising at least a first loop part and a second loop part, each loop part being coupled with a respective capacitor to define a first resonant frequency for the first loop part and a second resonant frequency for the second loop part, the resonant frequencies being different, and
the two loop parts being jointly connected to a shared input connection for receiving driving signals for driving both the loop parts;
a driving circuit connected to the input connection and adapted to drive the antenna at each of the resonant frequencies; and
a control means adapted to implement switching of the driving circuit between the two frequencies.

The device is effectively a signal generation device for generating electromagnetic signals for stimulating generation of eddy currents in the probed medium. When secondary electromagnetic signals, induced by the eddy currents, are returned to the antenna, these may be detected in some embodiments by means of coupled signal generation circuitry for example. Thus a signal generation device may be provided.

The invention is based on use of a dual-loop structure. Two different loop parts are provided, each configured with a different resonant frequency, but the two parts fed by a single input feed. This means that the antenna can be operated in the same way as if it consisted of only a single loop part, supplying a single drive line which feeds both loop parts. However, by simply varying the supply frequency between the two resonant frequencies, a different one of two loop parts can be induced to resonate. This means a single antenna is selectively capable of resonating at two different frequencies and hence capable of generating signals of comparative strength at two different frequencies. At the first frequency, the first loop part provides the major part of the signal strength contribution, at the second frequency, the second loop part provides the major part of the signal strength contribution. Each loop part thus effectively acts as a separate antenna (or separate resonator), but electrically coupled with a common drive signal input.

Hence, multi-frequency signal generation is possible without compromised field strength in one of the two frequencies.

The driving circuit is operable to provide the common driving signal at one or other of the resonant frequencies at a time.

By selectively switching the frequency of the common drive signal between the different resonance frequencies of the loop parts, this thereby switches which of the different loop parts is (primarily) active at a given time (i.e. which is oscillated at resonance).

The device finds advantageous application for sensing electromagnetic signals emitted from a medium in response to propagation into the medium of electromagnetic excitation signals. The excitation signals can be generated by resonating the antenna at one of the resonant frequencies. Holding the antenna in proximity to a medium, such as a body, the signals are inductively coupled into the medium, and induced electromagnetic response signals are (typically simultaneously) coupled back out. This mutual coupling induces changes in the electrical characteristics of the current in the coil which can be detected to derive properties of the stimulated medium.

The device comprises a control means. In some cases, the driving circuit itself may comprise the control means, i.e. the driving circuit may comprise an integrated control circuit or control unit or a processor for implementing the switching function.

The control means may be configured to implement switching of the driving circuit between the two resonant frequencies in accordance with a pre-defined control program or control schedule. This may be stored in a local or remote memory for example.

Examples discussed in this disclosure relate predominantly to example arrangements comprising two loop parts. However, further examples in accordance with the invention may comprise more than two loop parts (i.e. three or more loop parts), each comprising a respective capacitor defining a respective different resonant frequency for that part. This would hence permit the single antenna to operate at three or more different frequencies, with the number of frequencies expandable as desired.

Each of the three or more loop parts in this case would be similarly be connected to said shared input connection for receiving driving signals for driving all of the loop parts, and the control means in this case adapted to implement switching of the driving circuit between the three of more frequencies.

The first and second loop parts are electrically connected to a common input connection, for example conductively coupled to the common input connection.

Preferably, the two loop parts are connected in electrical parallel (to said shared input connection).

The drive circuit is electrically connected to the common input connection, for example conductively coupled to the common input connection, e.g. by a conductive line.

The resonant frequency of each loop part means the current frequency at which the loop is resonant.

Each loop part may be a closed loop part, i.e. each loop part forms or defines a respective closed loop. These respective closed loops are separate. The lines defining the different loop parts may however be partially formed by one or more shared loop sections.

Each loop part forms a separate circuit branch of the antenna (circuit). Both loop parts may be connected to a common ground.

Each loop part may be formed by a single turn loop. Keeping the number of windings small advantageously minimizes capacitive effects between the wires. However a single winding is not essential and one or both loop parts may comprise more windings in other examples.

Each loop part defines a separate respective interior area. The first and second loop parts may define or bound respective interior areas. The two loops may be arranged adjacent to one another for example. The interior area means the open inside region bounded by the loop.

The antenna may comprise at least one shared loop section which forms a portion of each loop part. Thus the two loop parts are joined by the shared loop section.

A terminal of the shared loop section may form the shared input connection of the antenna, for receiving the driving signals. By supplying the drive signals to the shared loop section, both loop parts are simultaneously supplied.

The first and second loop parts may each be formed by coaxial lines, i.e. each is formed by one or more loops of co-axial line. By line is meant the signal carrier line of the loop, e.g. wire. Each loop part in this case comprises an inner core line part and co-axially surrounding tubular outer line part. Alternating signals are driven between the inner core conductor part and the outer surrounding conductor part.

The control means may be adapted in at least one control mode to switch the driving circuit alternately between the two resonant frequencies.

The frequency of the switching can be adjusted as desired. In one set of examples, the frequency may be configured in dependence upon the duration of physiological phenomenon being monitored. In particular, it may be set sufficiently fast that the physiological process is captured by both loop parts. For example the period of the switching cycle (between both frequencies and back again) may be set to be less than the duration of the monitored phenomenon, and preferably less than half said duration, and more preferably less than one quarter of said duration.

For typical applications, a switching (sampling) frequency of between 4-200 Hz is suitable for ensuring the phenomenon is captured by both loop parts. Lower frequencies such as 4-20 Hz are useful for relative low-power (low-detail) applications, while 20-200 Hz is useful when monitoring at a higher time resolution, to resolve physiological waveform features at greater detail. To save power, optionally the control means may incorporate an idle time within the switching cycle, between the frequencies, during which none of the loop parts is operational.

The antenna may be configured such that the first and second loop parts occupy a common plane. The first and second loop parts may define respective interior areas which are non-overlapping in this case.

In other examples, the first and second loop parts may define respective interior areas which are overlapping. For instance one loop part may be inset within the other, for instance with the two joined to a common edge section which forms one portion (e.g. one side) of each loop part. This overlapping arrangement achieves equivalent performance in terms of inductive sensing capability as the non-overlapping arrangement, and provides the additional advantage of reduced-form factor.

The antenna may in either case thus be configured in a planar or flat configuration. This may achieved by forming the antenna from a printed circuit board (PCB) material for example using standing circuit printing techniques.

According to an advantageous set of embodiments, the device comprises a planar ground plate element electrically coupled with the antenna for grounding both loop parts of the antenna. The antenna is preferably separated from the ground plate by an insulating space or medium, for example air or a dielectric material.

The antenna may in this case have a planar configuration, and wherein the planar ground plate element is arranged in planar facing relationship with the planar antenna. The two are spatially separated by a space or clearance, or by an intermediate medium or layer. The two may be supported in separation for instance by a dielectric layer.

In this case or otherwise, the ground plate element may perform an electromagnetic shielding function.

The first and second loop parts may each define a respective interior area, and wherein the planar ground plate element comprises at least one opening through the plate element, the opening having a planar area which extends transversely across a major portion of one or both of said interior areas. The opening permits outward transmission of electromagnetic signals for coupling with a medium.

Opening means a hole: an opening extending through the plate from one planar side to the other. A major portion in this disclosure means a majority portion (more than half of or most of).

In one example, the planar ground plate element may comprise a single opening extending across major portions of both of said interior areas.

In an alternative example, the ground plate element may comprise two spaced openings, preferably each having an area extending across a major portion of a different respective one of said interior areas.

In either case, the shape of a given opening may be similar or substantially similar (in mathematical sense) to an outer perimeter of the antenna or to an outer perimeter of one of the loop parts of the antenna. The given opening has the same shape in this case as an outer or outline shape of the overall antenna or of one loop part of it.

Where a single opening is provided, the consequent antenna structure results in EM waves having components which propagate perpendicular to the plane of the plate (in the case of a planar antenna arranged parallel with the plate).

Where two openings are provided, the consequent antenna structure results in EM waves which propagate parallel to the plane of the plate.

The ground plate element may additionally or alternatively comprise a slot or gap extending from the outer edge of the ground plate, inward. It may extend from the outer edge of the plate to said opening or openings. It longitudinally traverses at least one section of the antenna line. There may be such gaps or slots provided traversing the line forming each of the loop parts of the antenna.

The sensing device may comprise signal processing means for deriving one or more signal characteristics associated with signals received at the antenna. This may be integrated with the control means in some examples.

The signal processing means permits deriving signal characteristics of signals coupled to the antenna from the medium. This may comprise in some cases sensing (changes in) electrical properties of the current running through the antenna line, e.g. frequency, amplitude, return losses.

The signal processing means may be adapted to derive one or more physiological parameters based on the sensed signal characteristics. These may for instance be one or more vital signs, including for example heart rate, pulse rate, breathing capacity, breathing rate, stroke volume, stroke volume variations, cardiac output, or aortic or arterial pulse height/pressure/diameter modulations.

The device may be a physiological inductive sensing device for sensing one or more physiological parameters of a body of a subject. The physiological parameters may include by way of example one or more of the vital signs noted above.

Examples in accordance with a further aspect of the invention provide a signal generation method, for use in inductive sensing, comprising coupling electromagnetic signals into a medium at two different frequencies, the method making use of an antenna, wherein the antenna comprises
at least a first loop part and a second loop part, each loop part being coupled with a respective capacitor to define a first resonant frequency for the first loop part and a second resonant frequency for the second loop part, the resonant frequencies being different, and
the two loop parts being jointly connected to a shared input connection for receiving driving signals for driving the both the loop parts;
and the method comprising
driving the antenna at each of the resonant frequencies, the driving comprising switching between the two resonant frequencies.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically illustrates stimulation of a thorax of a subject using a loop antenna;
Fig. 2 shows an example inductive sensing device according to an embodiment;
Figs. 3 and 4 show an example antenna and shielding arrangement for an example inductive sensing device according to an embodiment;
Figs. 5 and 6 show a further example antenna and shielding arrangement for a further example inductive sensing device according to an embodiment;
Figs. 7 and 8 show a further example antenna and shielding arrangement for a further example inductive sensing device according to an embodiment, the antenna having overlapping loop parts; and
Fig. 9 shows a block diagram of an example signal processing circuit.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an inductive sensing device for sensing at two different frequencies. The device includes an antenna which comprises at least two loop parts, each loop part coupled to a different respective tuning capacitor to thereby provide different first and second resonant frequencies for the first and second loop part respectively. The two loop parts are jointly connected to a shared input connection for receiving driving signals for driving both antenna parts. A control means controls a drive circuit to supply the antenna, via the shared input connection, drive signals of each of the frequencies, implementing switching between the two.

Embodiments of the present invention operate on the principle of inductive coupling, whereby a coil or wire has induced across it a potential difference due to exposure to a time-varying magnetic field. Embodiments of the present invention use this principle to measure strength of electromagnetic signals generated within regions of a medium or body by sensing changes in characteristics of a current generated in a loop antenna placed in proximity of the body. Changes in the inductance of the coil may be sensed, where these changes are detected for instance based on changing resonance characteristics of the coil circuit.

Embodiments of the invention may be used to sense electromagnetic signals emitted by a body in response to an electromagnetic excitation signal that is emitted into the body. In advantageous examples, the same antenna may be used to generate the excitation signals as to sense the returned electromagnetic signals.

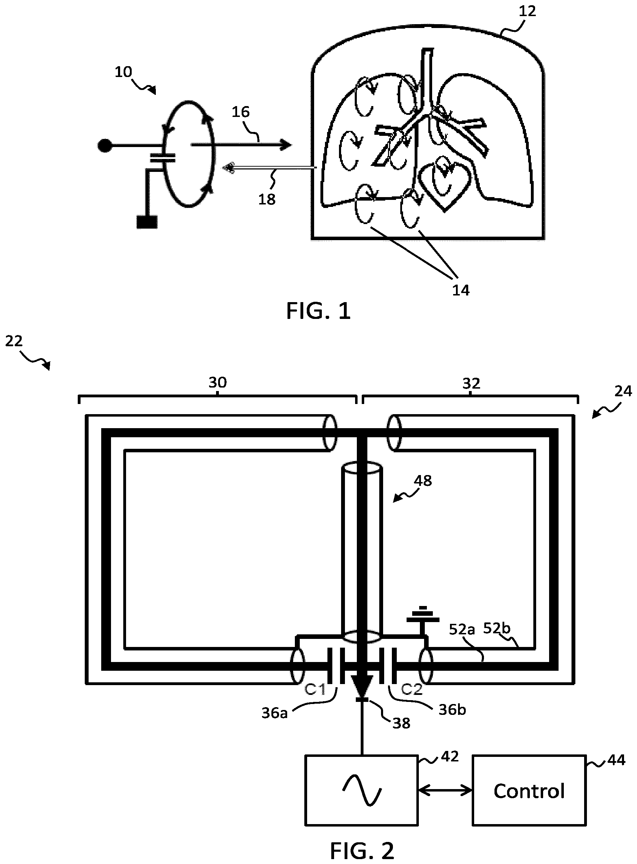

This principle is illustrated schematically in Fig. 1, which shows by way of example a loop antenna 10 (not in accordance with the invention) being driven with an alternating current in proximity to a thorax 12 of a subject, so as to propagate electromagnetic signals 16 into the thorax.

As a consequence, eddy currents 14 are induced within in the thorax. The eddy currents naturally arise due to Faraday's law of induction, whereby an electromotive force (EMF) is induced in a conducting medium in response to presence of a time-varying magnetic field.

The eddy currents in turn result in generation of a time-varying magnetic flux 18 of equivalent frequency to that generated by the primary antenna 10. These fluxes result in propagation of electromagnetic waves (signals) 18 which can be sensed at the antenna 10.

In accordance with advantageous examples, the device may be used for sensing physiological parameters and properties, for instance air, fluid and/or tissue movements in the body of a subject. The system may be advantageously applied in particular for sensing breathing movements for instance.

In these examples, the device can be used to sense air, fluid and/or tissue movements (e.g. caused by breathing or the beating of the heart) by sensing modulations in the reflected inductance of the signal caused by these movements.

It will be appreciated that movements of tissue in the body can comprise changes in volume of the tissue. These modulations cause amplitude and/or phase modulations of the electromagnetic signal.

A modulated electromagnetic signal is emitted by the body in response to the electromagnetic excitation signal that is emitted into the body of the subject. As the tissue, air or fluid move or expand and contract, the eddy currents generated in the medium change shape and form resulting in modulation of the returned EM signals. This allows sensing of changes and movement beneath the surface of a body.

It is of benefit to provide an inductive sensing device capable of performing sensing using inductive electromagnetic signals of more than one frequency, as different structures of the body (e.g. lungs, heart, skin) are more or less sensitive to different frequencies. It is a challenge to provide a simple inductive sensing arrangement capable of generating signals of more than one frequency, where the signals in both frequencies are of high strength.

The solution of the present invention provides a loop antenna design permitting generation of strong magnetic fields in both a low and high frequency range, to measure in-body eddy currents at two different frequencies, while retaining a single antenna input connection for driving signals.

Fig. 2 shows an example inductive sensing device according to an embodiment of the invention.

The device 22 comprises an antenna 24, the antenna having a first loop part 30 and a second loop part 32. Each loop part defines a distinct closed loop and forms a distinct branch of the antenna circuit. The first loop part is coupled with a first tuning capacitor 36a, of capacitance C1, defining a first resonant frequency, f₁, for the first loop part. The second loop part is coupled with a second capacitor 36b, of capacitance C2, defining a second resonant frequency, f₂, for the second loop part. The first and second resonant frequencies are different. This can be achieved by selecting C1 and C2 to be different from one another for example.

The two loop parts 30, 32 are jointly connected to a shared input connection 38 for receiving driving signals for driving both the loop parts. A common input drive signal drives both loop parts together. A driving circuit, in the form of an oscillator circuit 42, is electrically coupled to the shared input connection and operable to drive the antenna 24 at least at each of the resonant frequencies. The driving circuit is conductively coupled to the shared input connection in this example, for example by a connecting conductor line (e.g. wire).

There is further provided a control means in the form of a controller 44 operably coupled with the drive circuit 42, the controller adapted to implement switching of the driving circuit between the two resonant frequencies f₁, f₂. In this way the sensing device 22 is adapted to drive the antenna 24 sequentially at the two resonant frequencies. In further examples, the controller may be integrated with the driving circuit.

Since each loop part 30, 32 is configured with a different resonant frequency, each loop part effectively acts in this arrangement as a separate tuned antenna (or separate resonator circuit), but wherein the two are jointly connected to a common signal input feed 38. Simply by driving the antenna 24 at the first resonant frequency, f₁, or the second resonant frequency, f₂, the first or second loop part respectively is induced to resonate, thereby generating high strength magnetic inductive signals of that frequency for coupling into a medium of interest. Hence signals of two different frequencies can be generated at high magnetic field strength, while retaining a single-connection antenna design.

The first and second loop parts each are formed by co-axial lines according to the present example. The co-axial line comprises an inner conductor core 52a part co-axially surrounded by a tubular conductor shield 52b part. The core and shield parts are separated by a tubular insulating (dielectric) layer. Although co-axial lines are used for this example, this is not essential to the invention and alternative conducting lines may be used in other examples. Various alternatives will be apparent to the skilled person.

Both the first 30 and second 32 loops parts are connected to a common ground, in particular the outer shield part of the coaxial cable forming each loop part is connected to the common ground.

The number of windings of the antenna 24 is preferably kept small, to minimize capacitive effects between the wires. The first second loop parts 30, 32 are each advantageously formed in this example by a single turn loop. However a single winding is not essential and one or both loop parts may comprise more windings in other examples.

In this example, the antenna 24 comprises a shared loop section 48 which forms one portion of each of the loop parts. The loop parts are connected via the shared loop section.

The shared loop section 48 facilitates the common driving of two loop parts 30, 32, since drive signals can be supplied via the shared loop section, simultaneously feeding both loops. In particular, in this example the shared input connection 38 is provided by a terminal of the shared loop section 48. However, this is not essential for the invention. Other advantages of the shared loop section include reducing the overall internal resistance of the antenna, by avoiding duplication of the shared section in two separate loops.

As noted, in use the controller 44 controls switching of the oscillator circuit 42 between generating and inputting to the antenna 24 electrical signals of AC frequency f₁ (the first resonant frequency) and AC frequency f₂ (the second resonant frequency). The controller may control the oscillator to alternate between the two resonant frequencies. The alternating between the frequencies may be at a high frequency.

The frequency of the switching can be adjusted as desired. In one set of examples, the frequency may be configured in dependence upon the duration of the physiological phenomenon being monitored. In particular, it may be set sufficiently fast that the physiological process is captured by both loop parts. For example the period of the switching cycle (between both frequencies and back again) may be set to be less than the duration of the monitored phenomenon, and preferably less than half of said duration, and more preferably less than one quarter of said duration.

For typical applications, a switching (sampling) frequency of between 4-200 Hz is suitable for ensuring the phenomenon is captured by both loop parts. Lower frequencies such as 4-20 Hz are useful for relative low-power (low-detail) applications, while 20-200 Hz is useful when monitoring at a higher time resolution, to resolve physiological waveform features at greater detail. To save power, optionally the control means may incorporate an idle time within the switching cycle, between the frequencies, during which none of the loop parts is operational.

Alternatively, the controller 44 may control switching sequentially from one resonant frequency to the next without any regular alternation back again. Switching may be implemented by the controller in accordance with input commands from a user in some examples. A user input means may be provided to facilitate this. For example, the frequency may be set statically at one (e.g. optimal) frequency for one patient, and set at a different frequency for a different patient.

As noted, the first resonant frequency and second resonant frequency are different. The resonant frequency of each loop part 30, 32 depends on the circumference of the respective loop and on the capacitance of the respective capacitor 36a, 36b. Both parameters together define the resonant frequency. Hence, tuning each part to a desired resonant frequency may involve appropriately adjusting both parameters to achieve the given frequency. Where the circumference of both loop parts is the same, tuning can be achieved by selecting C1 and C2 to be different from one another for example.

For the example of Fig. 1, each loop part 30, 32 has a rectangular configuration. The shared loop section 48 provides one side of each of the rectangles. A rectangular shape is not essential and any other closed loop or annular shape may alternatively be used in further examples, for example rounded (regular or irregular), e.g. circular or oval, or triangular or any other loop shape. A regular shape is preferred, for generating symmetric electromagnetic field patterns.

The two loop parts may have the same shape or different shapes. The two loop parts may be the same size or different sizes, e.g. the same circumference or difference circumferences or define (bound) the same interior area or different interior areas.

According to a preferred set of embodiments, the antenna is configured in a flat or planar configuration, wherein the first and second loop parts occupy a common plane. This may be achieved for example by forming the antenna structure from printed circuit board (PCB) material using commonly used production techniques. This provides it a rigid structure.

In certain embodiments, the first and second loop parts each bound a respective interior area, wherein these areas are non-overlapping. This arrangement is shown in the example of Fig. 2 for example. However, in other examples, the first and second loop parts may each bound a respective interior area, where these areas do overlap. One loop may be inset inside the other for example.

As noted, the signal sensing device 22 comprises a driving circuit 42. The driving circuit may be or may comprise a signal generating means. In the example of Fig. 2, the driving circuit is in the form of an oscillator circuit. The oscillator is arranged to supply oscillatory drive signals to the common input connection 38 to excite the antenna 24 with an oscillatory current in order thereby, in use, to generate a sinusoidal electromagnetic signal (a sinusoidal electromagnetic wave) for propagation into the body to be stimulated.

Use of an oscillator circuit is not essential. In other examples a different form of drive circuit may be used, for example a drive circuit adapted to generate pulsed electrical signals having a pulse frequency corresponding to one of the loop part 30, 32 resonant frequencies f₁, f₂.

The sensing device 22 may further comprise signal processing means for analyzing signal characteristics of signals coupled into the antenna 24 from a medium being investigated. This is not essential the invention however, which is concerned primarily with improvement to the means for coupling signals into and out of a medium (in particular to facilitate coupling at two different frequencies), this being for use in performing inductive sensing. Signal processing may be performed for example by an external unit or device.

Where signal processing means are provided, the signal processing means may be provided in the form of a signal processing unit. Alternatively, in some examples, the signal generation (driving) and signal processing may be facilitated by a single unit. In accordance with particular examples, the functionality of both these elements may be facilitated by a Vector Network Analyzer, containing an oscillator for exciting the antenna 24 and impedance measurement means for sensing received signals.

In further examples, the signal processing means may be integrated with the control means, for example with the controller unit 44 in the present example.

The signal processing means analyses the characteristics of response signals received at the antenna 24 from a medium being excited by the antenna. In particular, the signal processing means may process received signals to derive a measure of changes in the damping factor and/or the resonant (or natural) frequency of the resonator circuit which is formed by each respective loop part 30, 32 of the antenna 24. When electromagnetic signals are received back at an antenna they detune the characteristics of the antenna circuit, and this allows the received signals to be measured.

A significant advantage of the antenna arrangement of the present invention is that regardless of which of the loop parts is being driven at resonance, the circuit properties of that antenna (and so the detuning characteristics of that antenna) can be directly measured from the output of the single feed point 38. This is because only one loop part is ever resonant at one time, meaning the output feed from the antenna is always dominated by the circuit properties exhibited in the resonant antenna. This makes detuning of the resonant circuit properties detectable regardless of which loop part is active.

The signal processing means may comprise a phase-locked loop (PLL) circuit according to one or more examples.

There may optionally be provided data communication means (not shown in Fig. 2) for facilitating communication between the controller 44 and an external device such as an external computer or data store. This may facilitate communication of signal processing results derived by a provided signal processing means to an external computer. It may also facilitate communication of control commands for instance to the controller from an external control means such as a computer.

The data communication means may comprise a wireless communication means or wired communication means. The communication means may implement or operate in accordance with any suitable communication protocol or medium such as for example Bluetooth, Wi-Fi, Near Field Communication (NFC), ZigBee, or any suitable wired communication protocol.

In use, the antenna 24 is held in proximity to a body or medium of interest, and the antenna is excited by the driving circuit 42 to generate electromagnetic excitation signals.

In accordance with advantageous examples, the inductive sensing device 22 may be used for sensing physiological parameters and properties, for instance air, fluid and/or tissue movements in the body of a subject. The system may be advantageously applied in particular for sensing breathing movements for instance.

In these examples, the device permits sensing of air, fluid and/or tissue movements (e.g. caused by breathing or the beating of the heart) by sensing modulations in the reflected inductance of the signal caused by these movements. This was explained with reference to Fig. 1 above.

The configuration of the antenna 24 of the system may take different forms.

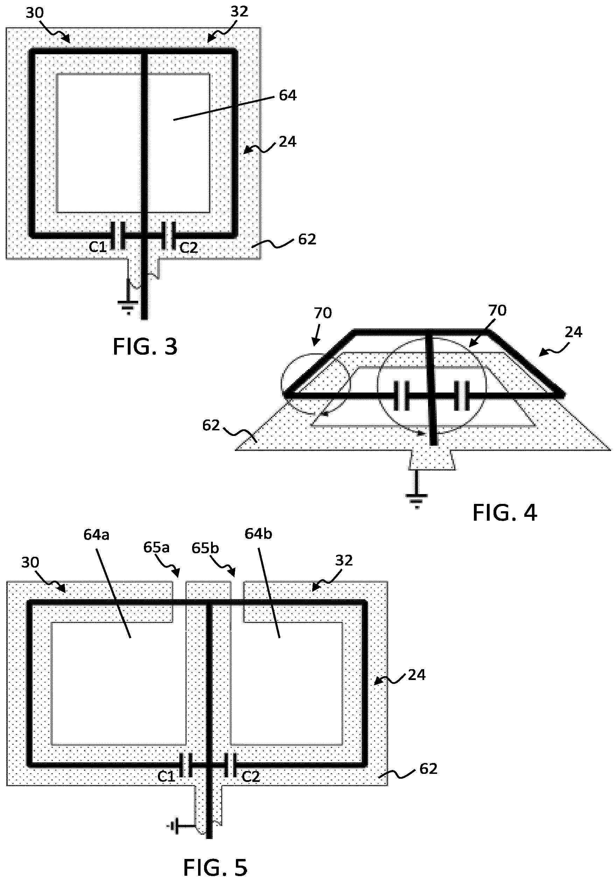

A set of examples comprising a planar antenna configuration and including electromagnetic shielding for the antenna will now be described with reference to Figs. 3-6.

Figs. 3 and 4 schematically depict a first flat-plane antenna 24 arrangement, wherein the sensing device 22 further includes a planar ground plate element 62 electrically coupled with the antenna for grounding both loop parts of the antenna. The electric coupling with the ground plate is purely capacitive: no connection line is required. The antenna may be connected to the ground plate via the outer shield part 30, 32. The antenna may be formed from PCB material to facilitate a stable, flat shape.

The planar ground plate element 62 delimits an opening 64 through the plate element. The opening has an extended planar area such that the opening effectively forms an open window area through the ground plate, or a missing portion of the ground plate. The opening extends from one planar side of the plate to the other. The (material of) the plate extends the whole way round the opening, effectively defining an annular shape around the opening, i.e. the opening is fully bounded or surrounded by the material of the plate.

In the example of Figs. 3 and 4, the planar ground plate 62 delimits a single opening 64. The opening has a shape which is similar or substantially similar (in a mathematical sense) to an outer perimeter of the antenna 24, i.e. the opening has the same shape in this case as an outer or outline shape of the overall antenna, but has an opening area which is smaller than the interior area bounded by the outer perimeter of the antenna.

The opening 64 is configured such that a planar area of the opening 64 extends transversely across a major portion of an interior area defined or bounded by each of the loop parts 30, 32 of the antenna 24. The opening is positioned such that (its planar area) maps spatially onto a central region of an interior area bounded by an outer perimeter of the antenna. In the particular example illustrated, an outer perimeter of the opening is concentrically aligned with an outer boundary of the antenna, such that the opening is centrally aligned within the interior area defined by the outer perimeter of the antenna.

The ground plate 62 acts as an electromagnetic shield (Faraday shield), shielding components of the antenna 24 from electromagnetic waves. The hole (opening) 64 permits propagation through the shield of magnetic field components both from and to the antenna.

The purpose of the (leaking) ground plate shield is to transfer the outgoing and, more importantly, incoming electromagnetic wave impedance, from high to low impedance, from electric field to magnetic field. In this way, in received signals, the electric field part is minimized as much as possible, as it is only the magnetic field part which contributes to the useful sensing signal. The electric field part merely generates noise in this sensing signal. Despite this, there always remains a residue of electric field; to block the electric components completely would also lead to blocking of the useful magnetic field parts.

In addition, the shield performs other functions. Due to its electrical coupling with the antenna, it provides a contribution the tuning each part of the loop. Furthermore, in examples, the shield may be arranged in such a way that it also shields the series capacitors 36a, 36b from incoming electric fields. Further, the shield may be arranged so as to reduce or prevent the build-up of electrical charges along the line of the antenna 24, which can thereby reduce the effect of capacitive coupling with the medium. Capacitive coupling with the medium is detrimental to performance, since it affects the magnetic sensing signal, and also leads to motion artefacts due to its sensitivity to the relative distance between the body and the antenna.

The positioning of the opening 64, having a planar area aligned with an interior area of the antenna, means that fields are still able to propagate across this antenna interior area, thus permitting inductive coupling of signals into the loops 30, 32. The same is true for signals generated by the antenna, which are induced within this interior region of the antenna, as illustrated in Fig. 4.

Fig. 4 shows a perspective view of the antenna 24 and ground plate (shield) 62 arrangement, and schematically depicts the generation of electromagnetic signals by the antenna, disposed above the ground plate in co-planar fashion, the two separated by a small clearance which may be filled with an insulating (i.e. dielectric) material or medium. The ground plate is thus arranged facing one planar side of the antenna. The magnetic field lines 70 of the generated signals are schematically illustrated.

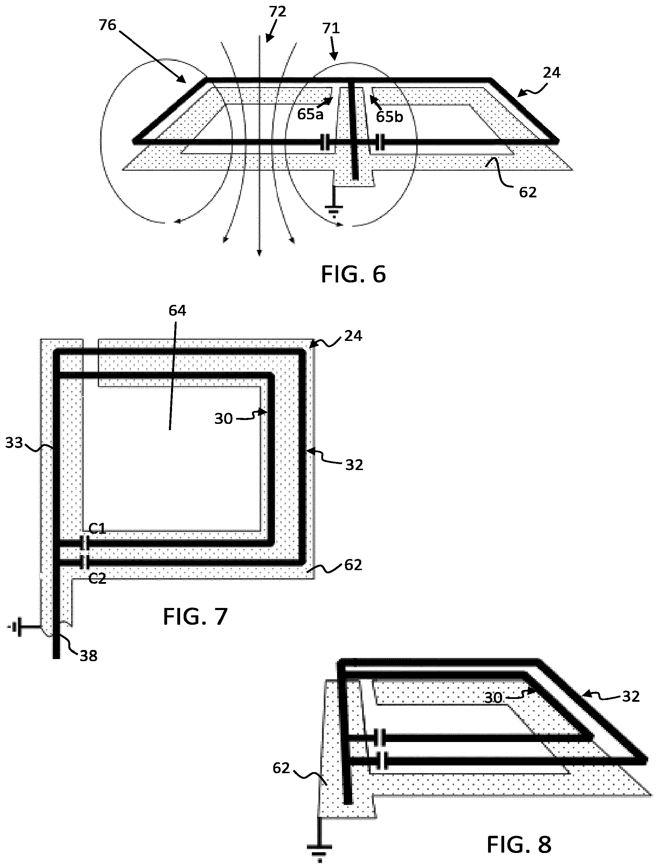

An alternative example configuration is illustrated in Figs. 5 and 6. This arrangement includes the same antenna 24 as in the arrangement of Figs. 3 and 4, but comprises a planar ground plate element 62 delimiting two openings 64a, 64b. The openings are the only difference between the ground plate of Figs. 5 and 6 and that of Figs. 3 and 4. Its electrical and structural properties, and its electrical interaction with the antenna (as a grounding element) are the same.

The planar ground plate element 62 delimits two openings 64a, 64b through the plate element, each opening again having an extended planar area so as to effectively form an open window area through the ground plate, or a missing portion of the ground plate. The two openings are spatially separated from one another. Each opening extends from one planar side of the plate to the other. The (material of) the ground plate 62 extends the whole way round each opening, effectively defining an annular shape around each opening, i.e. the openings are each fully bounded or surrounded by the material of the plate.

As noted above, the first and second loop parts 30, 32 of the antenna 24 each bound a respective interior area. The openings 64a, 64b are configured such that a planar area of each opening extends transversely across (or occupies) a major portion of a (different) respective one of said interior areas. The first opening 64a spans across a major portion of the interior area defined by the first loop part 30. The second opening spans 64b across a major portion of the interior area defined by the second loop part 32.

Each opening 64a, 64b is positioned such that (its planar area) maps spatially onto a central region of an interior area bounded by the outer perimeter of one of the respective loop parts 30, 32. In the particular example illustrated by Figs. 5 and 6, an outer boundary of each opening is concentrically aligned with an outer boundary of a respective one of the loop parts, such that each opening is centrally aligned within the interior area defined by this outer perimeter.

Each of the openings 64a, 64b has a shape which is similar or substantially similar (in a mathematical sense) to an outer perimeter of a different respective one of the loop parts 30, 32 of the antenna 24, i.e. the opening has the same shape in this case as this outer perimeter.

The planar ground plate element 62 optionally further delimits two slots or gaps 65a, 65b which extend longitudinally from an interior of each of said planar openings 64a, 64b in the ground plate to the edge of the plate. Each hence defines an open path from the interior of a respective one of the planar openings to the exterior of the ground plate. Each defines an interruption in the ground plate material. Each gap traverses (extends from one side to the other of) a line of one of the loop parts 30, 32.

The purpose of the gaps 65a, 65b is to block eddy-currents from forming in the annular-shaped sections of the ground plate which extend around each of the two planar openings 64a, 64b. The gaps hence form interruptions or breaks in each of these annular-shaped sections of the ground plate. It is advantageous to prevent the formation of such eddy currents (due to the propagation of back-reflected magnetic signals) in these annular sections, to avoid the consequent inducement of magnetic fields by the currents which are in opposition to those generated (or sensed) by each antenna loop part 30, 32.

Fig. 6 schematically illustrates the magnetic field pattern generated according to this arrangement. For simplicity, the field lines for only one loop part 30 are illustrated, corresponding to the first loop part 30 being driven at resonance. However, a similar, symmetrical pattern is created also for the second loop part 32 during times when it is driven at resonance. It can be seen that for the arrangement comprising two openings in the ground plate shield 62, both parallel 70 and perpendicular 72 magnetic field components are generated. The same circulatory-like field pattern 70 is generated circulating between the two loop parts (and also around the outer wing 76 of the antenna part), but in addition a parallel field component 72 induced, and able to propagate through a central region of the opening 64a aligned beneath it.

Although in the above described arrangements, electromagnetic shielding is provided on only one side of the antenna 24, in particular the side situated in use between the antenna and the medium to be investigated, in further examples shielding may be provided on both sides. According to one set of examples for instance, a continuous shielding plate (with no openings) may be provided on the alternate side of the antenna (the environment-facing side, away from the medium). In this way, the antenna is maximally shielded from environmentally originating electromagnetic signals, which could interfere with the coupling of signals of interest, originating from the medium being probed.

However, in further examples, the shielding on the alternate (environment-facing) side may comprise one or more openings. It may for example comprise the same configuration of openings as is provided for the shielding on the medium-facing side of the antenna.

In each of the above examples (Figs. 2-6), the antenna 24 is configured such that the two loop parts 30, 32 are non-overlapping with one another. In particular, the first and second loop parts each bound a respective interior area, wherein these areas are non-overlapping.

However, in other examples, the first and second loop parts may overlap with one another. An example is illustrated in Figs. 7 and 8. Here the first loop part 30 is inset inside the second loop part 32. Three sides of the first loop part extend concentrically with the corresponding sides of the second loop part 32, though concentricity is not essential. One side of each of the loop parts is formed by a common section 33 of antenna line. This common loop section 33 is connected to the shared input connection 38 for the two loop parts, to facilitate driving each of the loop parts.

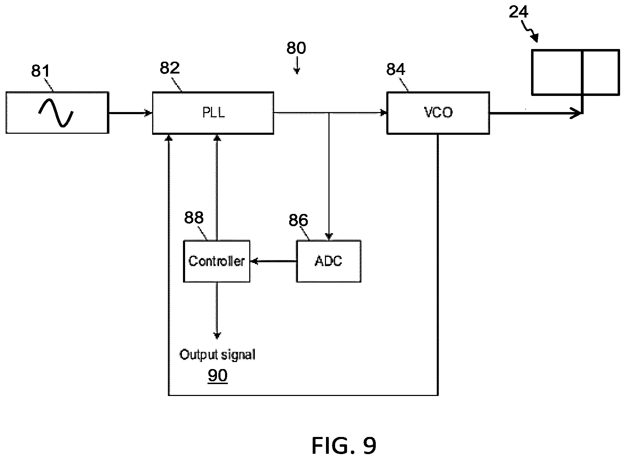

Simulations have found that the performance of the antenna 24 is not affected by overlapping of the loop parts 30, 32 in accordance with the arrangement illustrated in Fig. 7. Hence the overlapping loops arrangement provides equivalent performance in inductive sensing at two different frequencies as the separated loop arrangement of the examples described above. Furthermore the overlapping loops arrangement provides advantages in terms of reduced form factor, and so may be advantageous where space saving is a concern. As discussed, the inductive sensing device may optionally include signal processing means for analyzing received signals from a medium being investigated. By way of illustration of this feature, one example implementation of signal processing will now be described in detail, with reference to Fig. 9. This represents just example signal processing approach, and other approaches and apparatuses will be apparent to the person skilled in this field.

In accordance with the presently described example, the signal processing means is implemented by a phased locked loop. An example of a phased locked loop circuit which may be used is shown in Fig. 9.

In this embodiment, a phase locked loop (PLL) is used to drive an antenna circuit according to the invention 24, and a control signal for the PLL provides an output signal representing the movement of air, fluid and/or tissue in the body of the subject. Hence, the circuit of Fig. 9 implements the functionality of both the drive circuit 42 and optional signal processing means.

Fig. 9 shows signal generation and processing circuitry 80 for the antenna 24, and comprises a reference oscillator 81, a PLL 82 that is connected to the reference oscillator 81 and that outputs an analog control signal (known as Vtune) to a voltage-controlled oscillator (VCO) 84. The Vₜᵤₙₑ signal is a result of a comparison of the signal from the reference oscillator 81 to the signal from the VCO 84. In response to the PLL analog control signal the VCO 84 generates an excitation signal at a required frequency and provides this to the antenna 24 so that the antenna emits the electromagnetic excitation signal(s). As noted above the electromagnetic excitation signal will induce eddy currents in the body of the subject, and these eddy currents will induce a magnetic flux which is sensed by the antenna 24. This generated flux results in a reflected inductance component *Lᵣ* in the inductance of the antenna coil. This can be sensed via detuning of characteristics of the coil, in particular the damping and natural frequency of the coil.

The excitation signal is also provided to the PLL 82 as part of a feedback loop. The analog control signal from the PLL 82 is also provided to an analog-to-digital convertor (ADC) 86 which converts the analog control signal into a digital signal, and this digital signal is provided to a controller 88. The controller 88 determines a digital control signal for the PLL 82 and provides this to the PLL 82. As the skilled person will be aware, in a PLL system, if the phase of the VCO 84 differs from the phase of the reference oscillator 81, the digital control signal corrects the VCO phase.

Movements of air, fluid and/or tissue in the body effectively detune the characteristics of antenna 24 (because of the reflected inductance), and the digital control signal counters this detuning and corrects the phase of the VCO 84. The digital control signal therefore carries information regarding the movements of the air, fluid and/or tissue, and the controller 88 determines an output signal 90 from the digital control signal that represents or contains the information on the movements of air, fluid or tissue in the body of the subject. Although this output signal 90 does not carry the actual phase and amplitude information, the physiological characteristics (e.g. heart rate, breathing rate) are clearly observable.

The correction signal Vₜᵤₙₑ that is required to keep the VCO 84 at the required frequency is used to measure amplitude and/or phase shifts due to movements of air, fluid and/or tissue in the body of the subject. The phase shifts tend to dominate the amplitude changes. The PLL correction signal (the digital control signal output by the controller 88 derived from the analog PLL correction signal) is used to determine the output signal 90. For example the output signal 90 can correspond to the digital control signal with suitable filtering and/or down-sampling to improve the signal-to-noise ratio.

The output signal hence carries a signal representative of the received electromagnetic signals, derived based on changes in the natural resonance frequency of resonating loop part 30, 32 which is currently active, this being reflected in the correction signal. When the antenna is being driven at the resonant frequency, f₁, of the first loop part, the PLL will provide a correction signal that relates to the detuning of f₁. When driven at the resonant frequency f₂ of the second loop part, the PLL will provide a correction signal that relates to the detuning of f₂.

Embodiments of the invention outlines above and defined in the claims provide an inductive sensing device which permits sensing at multiple frequencies, each with a high field strength. Sensing at different frequencies permits selective tuning of the sensitivity of the system toward different structures of the body, such as the lungs, heart, and skin. For example, high-frequency measurements are predominantly sensitive to the skin (and therefore motion). Medium- and/or low-frequency measurements have optimized sensitivity to the heart and/or lungs (but also sensitive to motion). These two frequency measurement could therefore be used in combination to compensate the heart and lung measurements for motion artefacts. Furthermore, using multiple frequencies enables better identification of the individual contributions of the heart and lungs to the modulations in the signals.

In addition, measurements at multiple depths in the lung are possible using multiple frequencies. This for example may facilitate better estimation of the breathing depth of a subject. It may also permit determination of the location of an infection (accompanied by local lung edema).

As discussed above, embodiments make use of a controller 44. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A device (22), for use in inductive sensing, for inductively coupling electromagnetic signals (16, 18) into and out of a medium, the device comprising:
an antenna (24), the antenna comprising at least a first loop part (30) and a second loop part (32), each loop part being coupled with a respective capacitor (36a, 36b) to define a first resonant frequency for the first loop part and a second resonant frequency for the second loop part, the resonant frequencies being different,
the two loop parts being jointly connected to a shared input connection (38) for receiving a common driving signal for driving the loop parts;
a driving circuit (42) connected to the shared input connection and operable to provide the common driving signal selectively at each of the resonant frequencies to thereby selectively drive the antenna at one or other of the resonant frequencies; and
a control means (44) adapted to implement switching of the driving circuit between the two frequencies.

2. The device (22) as claimed in claim 1, wherein each loop part (30, 32) is formed by a single turn loop.

3. The device (22) as claimed in claim 1 or 2, wherein the antenna comprises at least one shared loop section (48) which forms a portion of each loop part.

4. The device (22) as claimed in any of claims 1-3, wherein a terminal of the shared loop section (48) forms said shared input connection (38) of the antenna (24).

5. The device (22) as claimed in any of claims 1-4, wherein the control means (44) is adapted in at least one control mode to switch the driving circuit (42) alternately between the two resonant frequencies.

6. The device (22) as claimed in any of claims 1-5, wherein the first (30) and second (32) loop parts define respective interior areas which are non-overlapping.

7. The device (22) as claimed in claim 6, wherein the antenna (24) is configured such that the first (30) and second (32) loop parts occupy a common plane.

8. The device (22) as claimed in any of claims 1-7, wherein the device comprises a planar ground plate element (62) electrically coupled with the antenna (24) for grounding both loop parts (30, 32) of the antenna.

9. The device (22) as claimed in claim 8, wherein the first (30) and second (32) loop parts each define a respective interior area, and wherein the planar ground plate element (62) delimits at least one opening (64) through the plate element, the opening having a planar area which extends transversely across a major portion of one or both of said interior areas.

10. The device (22) as claimed in claim 9, wherein the planar ground plate element (62) comprises a single opening (64) extending across major portions of both interior areas.

11. The device (22) as claimed in claim 9, wherein the ground plate element (62) comprises two spaced openings (64a, 64b), preferably each having an area extending across a major portion of a different respective one of said interior areas.

12. The device (22) as claimed in any of claims 8 -11, wherein the ground plate element (62) performs an electromagnetic shielding function.

13. The device (22) as claimed in any of claims 1-12, wherein the sensing device comprises signal processing means for deriving one or more signal characteristics associated with signals received at the antenna (24) .

14. The device (22) as claimed in any of claims 1-13, wherein the device is a physiological inductive sensing device for sensing one or more parameters of a body of a subject.

15. An inductive signal generation method comprising coupling electromagnetic signals into a medium at two different frequencies, the method making use of an antenna, wherein the antenna (24) comprises
at least a first loop part (30) and a second loop part (32), each loop part being coupled with a respective capacitor (36a, 36b) to define a first resonant frequency for the first loop part and a second resonant frequency for the second loop part, the resonant frequencies being different, and
the two loop parts being jointly connected to a shared input connection (38) for receiving driving signals for driving the both the loop parts;
and the method comprising
driving the antenna at each of the resonant frequencies, the driving comprising switching between the two resonant frequencies.

## Patentansprüche

1. Ein Gerät (22) zur Verwendung beim induktiven Abtasten, um elektromagnetische Signale (16, 18) in ein und aus einem Medium induktiv zu koppeln, wobei das Gerät Folgendes umfasst:
eine Antenne (24), wobei die Antenne mindestens einen ersten Schleifenteil (30) und einen zweiten Schleifenteil (32) umfasst, wobei die einzelnen Schleifenteile mit einem entsprechenden Kondensator (36a, 36b) verbunden sind, um eine erste Resonanzfrequenz für den ersten Schleifenteil und eine zweite Resonanzfrequenz für den zweiten Schleifenteil zu definieren, wobei sich die Resonanzfrequenzen unterscheiden,
und wobei die beiden Schleifenteile gemeinsam an einen gemeinsamen Eingangsanschluss (38) angeschlossen werden, um ein gemeinsames Ansteuerungssignal zum Ansteuern der Schleifenteile abzurufen;
ein Steuerkreis (42), der mit dem gemeinsamen Eingangsanschluss verbunden ist und das gemeinsame Ansteuerungssignal für die einzelnen Resonanzfrequenzen selektiv bereitstellt, um auf diese Weise die Antenne bei der jeweils einen oder anderen Resonanzfrequenz selektiv anzusteuern; und
eine Steuerung (44) zum Umschalten des Steuerkreises zwischen den beiden Frequenzen.

2. Das Gerät (22) gemäß Anspruch 1, wobei die einzelnen Schleifenteile (30, 32) mithilfe einer einfachen Schleife gebildet werden.

3. Das Gerät (22) gemäß Anspruch 1 oder 2, wobei die Antenne mindestens einen gemeinsamen Schleifenabschnitt (48) umfasst, der einen Teil der einzelnen Schleifenteile bildet.

4. Das Gerät (22) gemäß einem der Ansprüche 1 bis 3, wobei ein Anschluss des gemeinsamen Schleifenabschnitts (48) den gemeinsamen Eingangsanschluss (38) der Antenne (24) bildet.

5. Das Gerät (22) gemäß einem der Ansprüche 1 bis 4, wobei die Steuerung (44) in mindestens einem Steuermodus den Steuerkreis (42) abwechselnd zwischen den beiden Resonanzfrequenzen umschaltet.

6. Das Gerät (22) gemäß einem der Ansprüche 1 bis 5, wobei der erste (30) und der zweite (32) Schleifenteil jeweils sich nicht überlappende Innenbereiche definieren.

7. Das Gerät (22) gemäß Anspruch 6, wobei die Antenne (24) so konfiguriert ist, dass der erste (30) und der zweite (32) Schleifenteil eine gemeinsame Ebene einnehmen.

8. Das Gerät (22) gemäß einem der Ansprüche 1 bis 7, wobei das Gerät ein ebenes Erdungsplattenelement (62) umfasst, das elektrisch mit der Antenne (24) verbunden ist, um beide Schleifenteile (30, 32) der Antenne zu erden.

9. Das Gerät (22) gemäß Anspruch 8, wobei der erste (30) und der zweite (32) Schleifenteil jeweils einen Innenbereich definieren, und wobei das ebene Erdungsplattenelement (62) mindestens eine Öffnung (64) durch das Plattenelement begrenzt, und wobei die Öffnung einen ebenen Bereich aufweist, der sich quer über den Hauptteil mindestens einer der Innenbereiche erstreckt.

10. Das Gerät (22) gemäß Anspruch 9, wobei das ebene Erdungsplattenelement (62) eine einzelne Öffnung (64) aufweist, die sich über den größten Teil beider Innenbereiche erstreckt.

11. Das Gerät (22) gemäß Anspruch 9, wobei das Erdungsplattenelement (62) zwei voneinander beabstandete Öffnungen (64a, 64b) umfasst, die vorzugsweise jeweils einen Bereich aufweisen, der sich über den Hauptteil des jeweils anderen Innenbereichs erstreckt.

12. Das Gerät (22) gemäß einem der Ansprüche 8 bis 11, wobei das Erdungsplattenelement (62) eine elektromagnetische Abschirmfunktion bereitstellt.

13. Das Gerät (22) gemäß einem der Ansprüche 1 bis 12, wobei das Sensorgerät eine Vorrichtung für die Signalverarbeitung zum Ableiten mindestens einer Signalcharakteristik der von der Antenne (24) empfangenen Signale umfasst.

14. Das Gerät (22) gemäß einem der Ansprüche 1 bis 13, wobei es sich bei dem Gerät um ein physiologisches induktives Sensorgerät zum Erfassen mindestens eines Parameters des Körpers eines Patienten handelt.

15. Eine Methode zur induktiven Signalerzeugung, bei der elektromagnetische Signale mit zwei unterschiedlichen Frequenzen in einem Medium gekoppelt werden, wobei für die Methode eine Antenne verwendet wird, wobei die Antenne (24) mindestens einen ersten Schleifenteil (30) und einen zweiten Schleifenteil (32) umfasst, wobei die einzelnen Schleifenteile mit einem entsprechenden Kondensator (36a, 36b) verbunden sind, um eine erste Resonanzfrequenz für den ersten Schleifenteil und eine zweite Resonanzfrequenz für den zweiten Schleifenteil zu definieren,
wobei sich die Resonanzfrequenzen unterscheiden, und wobei die beiden Schleifenteile gemeinsam an einen gemeinsamen Eingangsanschluss (38) angeschlossen werden, um Ansteuerungssignale zum Ansteuern der beiden Schleifenteile abzurufen;
und wobei die Methode folgenden Schritt umfasst:
Ansteuern der Antenne bei beiden Resonanzfrequenzen, wobei das Ansteuern ein Umschalten zwischen den beiden Resonanzfrequenzen umfasst.

## Revendications

1. Dispositif (22), destiné à être utilisé dans la détection inductive, permettant de coupler par induction des signaux électromagnétiques (16, 18) dans et hors d'un support, le dispositif comprenant :
une antenne (24), l'antenne comprenant au moins une première partie boucle (30) et une seconde partie boucle (32), chaque partie boucle étant couplée à un condensateur (36a, 36b) respectif pour définir une première fréquence de résonance pour la première partie boucle et une seconde fréquence de résonance pour la seconde partie boucle, les fréquences de résonance étant différentes,
les deux parties de boucle étant connectées conjointement à une connexion d'entrée partagée (38) pour recevoir un signal d'attaque commun pour attaquer les parties de boucle ;
un circuit d'attaque (42) connecté à la connexion d'entrée partagée et pouvant fonctionner pour fournir le signal d'attaque commun sélectivement à chacune des fréquences de résonance pour ainsi attaquer sélectivement l'antenne à l'une ou l'autre des fréquences de résonance ; et
un moyen de commande (44) conçu pour réaliser la commutation du circuit d'attaque entre les deux fréquences.

2. Dispositif (22) selon la revendication 1, dans lequel chaque partie de boucle (30, 32) est formée par une boucle à une seule spire.

3. Dispositif (22) selon la revendication 1 ou 2, dans lequel l'antenne comprend au moins une section de boucle partagée (48), laquelle forme une partie de chaque partie de boucle.

4. Dispositif (22) selon l'une quelconque des revendications 1 à 3, dans lequel une borne de la section de boucle partagée (48) forme ladite connexion d'entrée partagée (38) de l'antenne (24).

5. Dispositif (22) selon l'une quelconque des revendications 1 à 4, dans lequel le moyen de commande (44) est conçu, dans au moins un mode de commande, pour commuter le circuit de commande (42) alternativement entre les deux fréquences de résonance.

6. Dispositif (22) selon l'une quelconque des revendications 1 à 5, dans lequel les première (30) et seconde (32) parties de boucle définissent des zones intérieures respectives, lesquelles ne se chevauchent pas.

7. Dispositif (22) selon la revendication 6, dans lequel l'antenne (24) est conçue de telle sorte que les première (30) et seconde (32) parties de boucle occupent un plan commun.

8. Dispositif (22) selon l'une quelconque des revendications 1 à 7, dans lequel le dispositif comprend un élément de plaque de masse (62) planaire couplé électriquement à l'antenne (24) pour mettre à la masse les deux parties de boucle (30, 32) de l'antenne.

9. Dispositif (22) selon la revendication 8, dans lequel les première (30) et seconde (32) parties de boucle définissent respectivement une zone intérieure respective, et dans lequel l'élément de plaque de masse (62) planaire délimite au moins une ouverture (64) à travers l'élément de plaque, l'ouverture comportant une zone plane, laquelle s'étend transversalement à travers une majeure partie d'une ou des deux zones intérieures.

10. Dispositif (22) selon la revendication 9, dans lequel l'élément de plaque de masse (62) planaire comprend une seule ouverture (64) s'étendant à travers des parties principales des deux zones intérieures.

11. Dispositif (22) selon la revendication 9, dans lequel l'élément de plaque de masse (62) comprend deux ouvertures (64a, 64b) espacées comportant respectivement, de préférence une zone s'étendant à travers une majeure partie d'une zone différente respective desdites zones intérieures.

12. Dispositif (22) selon l'une quelconque des revendications 8 à 11, dans lequel l'élément de plaque de masse (62) remplit une fonction de blindage électromagnétique.

13. Dispositif (22) selon l'une quelconque des revendications 1 à 12, dans lequel le dispositif de détection comprend des moyens de traitement de signal permettant de dériver au moins une caractéristique de signal associée aux signaux reçus au niveau de l'antenne (24).

14. Dispositif (22) selon l'une quelconque des revendications 1 à 13, dans lequel le dispositif est un dégivreur de détection inductif physiologique permettant de détecter au moins un paramètre d'un corps d'un sujet.

15. Procédé de génération des signaux inductifs comprenant le couplage des signaux électromagnétiques dans un milieu à deux fréquences différentes, le procédé utilisant une antenne, dans lequel l'antenne (24) comprend
au moins une première partie boucle (30) et une seconde partie boucle (32), chaque partie boucle étant couplée à un condensateur (36a, 36b) respectif pour définir une première fréquence de résonance pour la première partie boucle et une seconde fréquence de résonance pour la seconde partie de boucle, les fréquences de résonance étant différentes, et
les deux parties de boucle étant connectées conjointement à une connexion d'entrée partagée (38) pour recevoir des signaux d'attaque pour attaquer les deux parties de boucle ; et
le procédé comprenant
la commande de l'antenne à chacune des fréquences de résonance, la commande comprenant la commutation entre les deux fréquences de résonance.
